# EUROPEAN PATENT SPECIFICATION

(11) **EP 1 644 055 B1**
(45) Date of publication and mention of the grant of the patent: **24.10.2007**
(21) Application number: 04774821.5
(22) Date of filing: 15.07.2004
(51) Int. Cl.: A61L 27/52

(54) **Fibre-reinforced polymer hydrogel material for cartilage replacement**
Faserverstärktes polymeres Hydrogelmaterial zum Ersatz von Knorpelgeweben
Matériau sous forme d'hydrogel polymère pour la réparation cartilagineuse

(30) Priority: 15.07.2003 NL 1023924
(43) Date of publication of application: 12.04.2006
(73) Proprietor: Stichting voor de Technische Wetenschappen, 3527 JP Utrecht (NL)
(72) Inventor: WIJLAARS, Marcel, NL-5921 EB Venlo (NL); HUYGHE, Jacques, Marie, René, Jan, B-2340 Beerse (BE); VAN DONKELAAR, Corrinus, Cornelis, NL-5641 HJ Eindhoven (NL)
(74) Representative: van Westenbrugge, Andries
(86) International application number: PCT/NL2004/000511
(87) International publication number: WO 2005/004943

(56) References cited:
- US-A1- 2002 022 884
- US-A1- 2002 048 595
- YOUNG C-D ET AL: "High-strength, ultra-thin and fiber-reinforced pHEMA artificial skin" BIOMATERIALS, ELSEVIER SCIENCE PUBLISHERS BV., BARKING, GB, vol. 19, no. 19, October 1998 (1998-10), pages 1745-1752, XP004161446 ISSN: 0142-9612
- CORKHILL P H ET AL: "THE POTENTIAL OF HYDROGELS AS SYNTHETIC ARTICULAR CARTILAGE" PROCEEDINGS OF THE INSTITUTION OF MECHANICAL ENGINEERS. JOURNAL OF ENGINEERING IN MEDICINE. PART H, MECHANICAL ENGINEERING PUBLICATIONS LTD, LONDON, GB, vol. 204, no. H03 PART H, 1990, pages 147-155, XP000169580 ISSN: 0954-4119

## Description

The present invention relates to a tissue substitute material, comprising a fibre-reinforced polymerised hydrogel. A tissue substitute material of this type is disclosed in: "High-strength, ultra-thin and fiber-reinforced pHE-MA artificial skin," in Biomaterials 19 (1998) pp. 1745-1752. This publication discloses a skin substitute material consisting of a hydrogel that has been reinforced with fibres, such as Spandex fibres.

The document US 2002/0022884 A discloses a surgical implant comprising a hydrogel reinforced by a three-dimensional mesh having sufficient strength and durability to be used as a replacement for a damaged meniscus.

For replacement of cartilage-like tissue, such as, for example, the intervertebral disc or joint cartilage, the swelling behaviour of the abovementioned tissue substitute material is relatively weak and the strength and toughness are inadequate. When put into the body, the properties of these tissues that are inherent to the body are not sufficiently simulated. This applies in particular in respect of the characteristic of these cartilage-like tissues of being able to swell when the composition of the fluid (water/salt) surrounding the tissues changes. Consequently, optimum adaptation to the environment cannot be provided.

The aim of the present invention is to provide a tissue substitute material that does have these properties, that is to say does have an ability to swell, it furthermore being important that the material has adequate strength and is relatively compliant.

Said aim is achieved with a tissue substitute material as described above in that the tissue substitute material simulates cartilage-like tissue and contains 10 - 70 % (m/m) fibres (based on the dry matter) and in that 1 - 5 % (m/m) (based on the dry matter) of a substance that contains ionised groups has been added to said hydrogel.

The ionised groups provide a Donnan osmotic pressure in the hydrogel that pretensions the fibres. This phenomenon is analogous to the Donnan osmotic pressure of the ionised glycosaminoglycans in cartilage-like tissue that pretensions the collagen fibres in the tissue. The choice of a relatively weak fibre ensures that forces along the hydrogel-fibre interface are sufficiently low. The tougher mechanical properties can be obtained by adding much more fibre compared with the known state of the art. However, in this context it is important that the swelling ability is retained and this is achieved by adding a substance that contains ionised groups. One example of such a substance is sodium methacrylate. The diameter and the form thereof are chosen depending on the properties desired. The form can be, for example, knitted, wound, chopped fibres or non-woven. A diameter of 20 µm is given as an example. The length of the fibre can range from millimetres to kilometres.

An example of such a fibre is a fibre based on polyurethane. Lycra (Dupont de Nemours) and Spandex are examples of such fibres. It has been found that such materials are not hydrophilic for pure water. However, in combination with one or more monomers that create the hydrogel matrix on polymerisation hydrophilic properties are obtained and in combination with the particularly elastic behaviour thereof a particularly robust bond to the matrix is obtained.

The volume of the fibre increases (up to 50 %) as a result of the penetration of the monomer solution into the fibre. As a result of the use of such fibres the mechanical properties and more particularly the elastic properties can be improved to such an extent that the properties of cartilage are more closely approached.

The monomers from which the hydrogel can be obtained by polymerisation can comprise HEMA (hydroxyethyl methacrylate) and/or sodium methacrylate. Other monomers, optionally in combination with one another and with one of the substances mentioned above, are also possible. The hydrophilic character of these monomers can be either based on adsorption or based on electrostatic attraction of hydrophilic cations by a fixed charge.

It has been found that a tissue substitute material obtained in this way has properties that approach the properties of cartilage or are even better than these. An appreciable percentage of pre-stretch is possible, whilst a high compressive strength (of the order of 10 MPa) is achievable. The tissue substitute material obtained in this way is compressible under long-term loading. The material is not compressible under short-term peak loads.

Polymerisation can be achieved with the aid of a chemical initiator, thermal initiation and/or initiation with the aid of light (UV).

It has been found that the tissue substitute material thus obtained swells to a greater or lesser extent depending on the conditions of the fluid surrounding it. For instance, it is possible to immerse the material in a solution with a relatively high salt concentration, as a result of which this material assumes a relatively small volume. After putting into, for example, a living being, the salt concentration will fall and more water will be absorbed, as a result of which the volume of the tissue substitute material increases and clasping is provided between the parts in which this tissue substitute material is used.

The amount of fibre compared with the polymerised hydrogel will be chosen depending on the desired strength. A value of approximately 60 % of the dry weight of fibre material is mentioned as an example.

The tissue substitute material described above can be produced by immersing the polyurethane-based fibre in an aqueous solution of one or more of the abovementioned monomers. The monomer solution is sucked up during immersion. The strength and rigidity of the material can be promoted by orienting the fibres. The polymerisation described above then takes place. It is possible to provide the fibres with an orientation during immersion or, respectively, winding or knitting, so as to be able to influence the properties of the tissue substitute material in various directions.

From preliminary studies it is anticipated that the tissue substitute material produced in the manner described above is biocompatible.

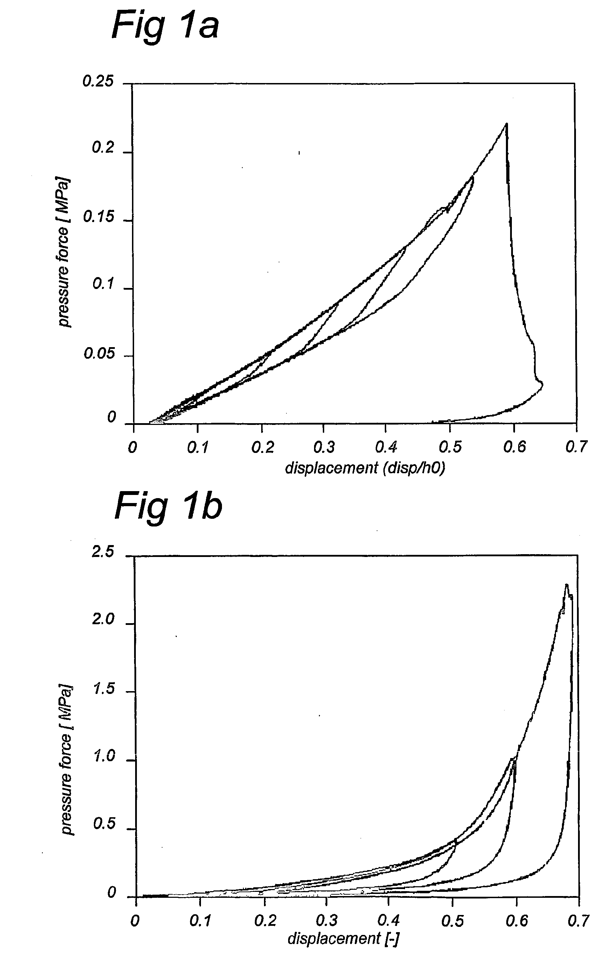

In Figure 1 a comparison is made between the mechanical properties on compression of a hydrogel sphere without fibre reinforcement and a hydrogel sphere in which the outer millimetre has been reinforced according to the invention with 60 % (m/m) (based on the dry matter) fibres. The hydrogel without fibre reinforcement is shown in Figure la and the hydrogel with fiber reinforcement is shown in Figure 1b.

Although the invention has been described above on the basis of particular embodiments, obvious variants that fall within the scope of the appended claims will be apparent to those skilled in the art after reading the above.

## Claims

1. Material for cartilage-like material substitution comprising a fibre-reinforced from monomers polymerised hydrogel, wherein this contains 10 - 70 % (m/m) swellable fibres (based on the dry matter) and wherein 1 - 5 % (m/m) (based on the dry matter) of a substance that contains ionised groups has been added to said hydrogel.

2. Material for cartilage-like material substitution according to Claim 1, wherein said hydrogel comprises a polymer based on hydroxyethyl methacrylate (HEMA).

3. Material for cartilage-like material substitution according to Claim 1 or 2, wherein said substance containing ionised groups comprises methacrylic acid.

4. Material for cartilage-like material substitution according to Claim 3, comprising 1 - 5 % (m/m) methacrylate.

5. Material for cartilage-like material substitution according to one of the preceding claims, wherein said fibre comprises a fibre saturated in liquid.

6. Material for cartilage-like material substitution according to one of the preceding claims, wherein said fibre comprises a fibre based on polyurethane material.

7. Prosthesis consisting of a material for cartilage-like material substitution according to one of the preceding claims.

## Patentansprüche

1. Material zum Ersatz knorpelähnlichen Materials mit einem faserverstärkten, aus Monomeren polymerisierten Hydrogel, welches 10 bis 70 % (m/m) quellfähige Fasern (auf Basis der Trockenmasse) enthält und wobei 1 bis 5 % (m/m) (auf Basis der Trockenmasse) einer ionisierenden Gruppen enthaltenden Substanz dem Hydrogel beigefügt wurde.

2. Material zum Ersatz knorpelähnlichen Materials nach Anspruch 1, wobei das Hydrogel ein Polymer auf Basis von Hydroxyethylmethacrylat (HEMA) umfasst.

3. Material zum Ersatz knorpelähnlichen Materials nach Anspruch 1 oder 2, wobei die die ionisierenden Gruppen enthaltende Substanz Methacrylsäure umfasst.

4. Material zum Ersatz knorpelähnlichen Materials nach Anspruch 3, umfassend 1 bis 5 % (m/m) Methacrylat.

5. Material zum Ersatz knorpelähnlichen Materials nach einem der vorangehenden Ansprüche, wobei die Faser eine in Flüssigkeit gesättigte Faser umfasst.

6. Material zum Ersatz knorpelähnlichen Materials nach einem der vorangehenden Ansprüche, wobei die Faser eine auf einem Polyurethanmaterial basierende Faser umfasst.

7. Prothese aus einem Material zum Ersatz knorpelähnlichen Materials nach einem der vorangehenden Ansprüche.

## Revendications

1. Matériau pour la substitution de matériau de type cartilage comprenant un hydrogel renforcé de fibre, polymérisé à partir de monomères, dans lequel celui-ci contient 10 à 70 % (m/m) de fibres gonflables (sur la base de la matière sèche) et dans lequel 1 à 5 % (m/m) (sur la base de la matière sèche) d'une substance qui contient des groupes ionisés ont été ajoutés audit hydrogel.

2. Matériau pour la substitution de matériau de type cartilage selon la revendication 1, dans lequel ledit hydrogel comprend un polymère à base de méthacrylate d'hydroxyéthyle (HEMA).

3. Matériau pour la substitution de matériau de type cartilage selon la revendication 1 ou 2, dans lequel ladite substance contenant des groupes ionisés comprend de l'acide méthacrylique.

4. Matériau pour la substitution de matériau de type cartilage selon la revendication 3, comprenant 1 à 5 % (m/m) de méthacrylate.

5. Matériau pour la substitution de matériau de type cartilage selon l'une des revendications précédentes, dans lequel ladite fibre comprend une fibre saturée en liquide.

6. Matériau pour la substitution de matériau de type cartilage selon l'une des revendications précédentes, dans lequel ladite fibre comprend une fibre à base de matériau poly(uréthane).

7. Prothèse consistant en un matériau pour la substitution de matériau de type cartilage selon l'une des revendications précédentes.
